# EUROPÄISCHE PATENTSCHRIFT

(11) **EP 2 765 825 B1**
(45) Veröffentlichungstag und Bekanntmachung des Hinweises auf die Patenterteilung: **22.04.2015**
(21) Anmeldenummer: 13154381.1
(22) Anmeldetag: 07.02.2013
(51) Int. Cl.: H05B 1/02, H05B 3/22, A61M 5/44

(54) **Fluidwärmer und Verfahren zum Betrieb eines Fluidwärmers**
Fluid warmer and method for operating same
Dispositif de chauffage de fluide et procédé de fonctionnement d'un dispositif de chauffage de fluide

(43) Veröffentlichungstag der Anmeldung: 13.08.2014
(73) Patentinhaber: Stihler Electronic GmbH, 70597 Stuttgart (DE)
(72) Erfinder: Stihler, Axel, 70597 Stuttgart (DE)
(74) Vertreter: Kohler Schmid Möbus Patentanwälte

(56) Entgegenhaltungen:
- DE-A1-102010 036 295
- GB-A- 2 052 109
- US-A- 5 381 510

## Beschreibung

Die Erfindung betrifft einen Fluidwärmer und ein Verfahren zum Betrieb eines Fluidwärmers.

Die Applikation größerer Mengen ungewärmter Fluide, beispielsweise beim intravenösen Verabreichen von Blutprodukten oder Infusionslösungen, kann nicht zuletzt aufgrund des damit einhergehenden Verlusts an Körperwärme nachteilige physiologische Effekte haben.

In der klinischen Praxis werden daher routinemäßig Fluidwärmer eingesetzt, um das in der Fluidleitung strömend geführte medizinische Fluid auf eine Temperatur nahe der Körpertemperatur von üblicher Weise 37° Celsius oder auch darüber vorzuwärmen. Die Fluidwärmer müssen in der klinischen Praxis über eine hohe Wärmeleistung verfügen, um auch medizinische Fluide bei höheren Flussraten zuverlässig aufzuwärmen. Die Fluidwärmer sollen dabei eine kompakte Bauform aufweisen, um deren Handhabung, insbesondere als mobile Einheiten oder auch als Anbaueinheiten für andere medizinische Geräte bzw. Apparate, zu vereinfachen. Am Markt verfügbare Fluidwärmer weisen deshalb in der Regel Widerstandsheizelemente für den Betrieb an Wechsel- bzw. Netzspannung auf, bei denen sich entsprechend dimensionierte Leistungsnetzteile erübrigen. Die Widerstandsheizelemente sind jeweils mit einem ersten und einem zweiten elektrischen Netzanschlussleiter versehen, um die Widerstandsheizelemente mit einer Wechselspannungsquelle (Netzspannung) elektrisch leitend zu verbinden. Zur Wärmeübertragung auf die Fluidleitung und das darin geführte medizinische Fluid dient ein Wärmeübertrager, der mit den Widerstandsheizelementen thermisch leitend verbunden (gekoppelt) ist. Die Fluidleitung weist aus Effizienzgründen in der Regel einen beutelförmigen Leitungsabschnitt auf, um eine effiziente Wärmeübertragung auf das Fluid zu gewährleisten. Der Wärmeübertrager ist häufig zumindest abschnittsweise plattenförmig ausgebildet, um eine effiziente Wärmeübertragung auf die Fluidleitung zu gewährleisten.

Die temperierten Fluide kommen in der Praxis typischer Weise unter Umgehung des Hautwiderstands mit der Person in einen elektrisch leitenden Kontakt. An Wechselspannung, d.h., insbesondere Netzspannung mit 100 V bis 240 V, betriebene Widerstandsheizelemente können aufgrund einer kapazitiven Kopplung mit anderen Bauteilen des Fluidwärmers, insbesondere dem Wärmeübertrager, zu Störungen anderer Diagnostik- bzw. Therapiegeräte, beispielsweise von EKG-Geräten, oder auch von elektrisch erregbaren Organstrukturen, insbesondere des Herzens, führen. Die im medizinischen Bereich eingesetzten Fluidwärmer müssen daher grundsätzlich hohen Anforderungen an die elektrische Sicherheit genügen und die strengen Sicherheitsnormen der international einheitlichen Klassifizierung "CF" (= Kurzform für "cardiac floating") erfüllen. Hiernach dürfen Patientenableitströme, d.h., über eine mit dem Fluid in Kontakt stehende Person geführte Leckströme, eine Stromstärke von insgesamt 10 µA (Mikroampere) unter Normalbedingungen und 50 µA im Fehlerfall nicht überschreiten.

Ein bekannter Fluidwärmer mit zwei Widerstandsheizelementen ist aus DE 10 2010 036 295 A1 bekannt.

Es ist Aufgabe der Erfindung einen eingangs genannten Fluidwärmer anzugeben, der die CF-Klassifikation bei einfachem konstruktivem Aufbau erfüllen kann. Darüber hinaus soll ein Verfahren zum Betrieb eines solchen Fluidwärmers angegeben werden, durch das ein nochmals höheres Maß an elektrischer Betriebssicherheit gewährleistet werden kann.

Die den Fluidwärmer betreffende Aufgabe wird durch einen Fluidwärmer mit den in Patentanspruch 1 angegebenen Merkmalen und die das Verfahren betreffende Aufgabe durch ein Betriebsverfahren mit den in Patentanspruch 12 angegebenen Merkmalen gelöst.

Weitere Vorteile und vorteilhafte Ausgestaltungen des Gegenstands der Erfindung ergeben sich aus der Beschreibung, den Ansprüchen und der Zeichnung.

Der erfindungsgemäße Fluidwärmer umfasst ein erstes und ein zweites Widerstandsheizelement für Wechselspannung und einen mit den beiden Widerstandsheizelementen gekoppelten Wärmeübertrager, der eine Aufnahme für die Fluidleitung aufweist. Jedes Widerstandsheizelement weist jeweils einen ersten und einen zweiten elektrischen Anschlussleiter zum Anschließen des Widerstandsheizelements an eine Wechselspannungsquelle auf. Die Anschlussleiter der beiden Widerstandsheizelemente sind jeweils mit einem ersten Schalter versehen, um die Widerstandsheizelemente mit der Wechselspannungsquelle stromführend zu verbinden oder aber von der Wechselspannungsquelle vollständig elektrisch zu trennen, d.h., einen Stromfluss über die Anschlussleiter der Widerstandsheizelemente insgesamt zu unterbinden.

Erfindungsgemäß können an den Widerstandsheizelementen des Fluidwärmers im Temperierbetrieb des Fluidwärmers zueinander jeweils gegenphasige Wechselspannungen anliegen. Die beiden Widerstandsheizelemente sind somit als ohmsche Verbraucher im Temperierbetrieb von Heizströmen durchflossen, die einen zueinander gegenphasigen zeitlichen Verlauf aufweisen. Dadurch können sich Störspannungen im Wärmeübertrager durch kapazitive Einstreuungen der beiden Widerstandsheizelemente in den Wärmeübertrager gegeneinander aufheben oder im Wesentlichen gegeneinander aufheben. Es versteht sich, dass die Widerstandsheizelemente dazu vorzugsweise übereinstimmende elektrische Kennwerte aufweisen und in einer vergleichbaren Position/Lage relativ zum Wärmeübertrager angeordnet sind. Zu beachten ist, dass es bei dieser Lösung freilich einer geräteinternen Wechselspannungsquelle bedarf, die für das Heizen des einen Widerstandsheizelement eine Wechselspannung bereitstellt, die zu der Wechselspannung des anderen Widerstandsheizelements exakt oder nahezu exakt um 180 ° phasenverschoben ist.

Die Widerstandsheizelemente können im Temperierbetrieb erfindungsgemäß auch mit zueinander gleichphasigen Wechselspannungen beaufschlagt sein, d.h., an den Widerstandsheizelementen liegen im Temperierbetrieb zueinander gleichphasige Wechselspannungen an. Die Widerstandsheizelemente sind in der Folge im Temperierbetrieb von Heizströmen durchflossen, die einen zueinander gleichphasigen zeitlichen Verlauf aufweisen. Der Fluidwärmer weist in diesem Fall zur Kompensation der kapazitiven Einstreuung der Widerstandsheizelemente in den Wärmeübertrager eine Kompensationsschaltung auf, durch die an dem Wärmeübertrager eine im Wesentlichen zur Wechselspannung der Widerstandsheizelemente gegenphasige Kompensationswechselspannung anlegbar ist. Von der Kompensationsschaltung muss dabei vorteilhaft nur diejenige Leistung erbracht werden, die tatsächlich durch die Einstreuungen in den Wärmeübertrager generiert wird. Bei einem Fluidwärmer mit beispielsweise 400 W elektrischer Leistung und einer kapazitiven Streuung (Ableitstrom) von beispielsweise 88 µA ergibt sich dann eine aufzuwendende Kompensationsleistung P mit P = 230 VAC • 88 µA = 0,02 W.

Bei beiden vorgenannten elektrischen Betriebsarten der Widerstandsheizelemente kann die mit dem Temperierbetrieb der Widerstandsheizelemente einhergehende kapazitive Einstreuung in den Wärmeübertrager und demgemäß in die Fluidleitung bzw. das darin geführte medizinische Fluid kompensiert oder im Wesentlichen kompensiert werden.

Der Fluidwärmer weist nach der Erfindung weiterhin eine Messeinrichtung zum Bestimmen einer Spannung zwischen dem Wärmeübertrager und einem Neutralleiter der Wechselspannungsquelle bzw. einem mit dem Neutralleiter elektrisch leitend verbundenen Bauteil des Fluidwärmers auf. Die Messeinrichtung ist dabei vorzugsweise für ein kontinuierliches Bestimmen bzw. Messen der Spannung ausgelegt.

Der Fluidwärmer weist erfindungsgemäß weiterhin eine Steuereinrichtung zum Steuern der Betriebsabläufe des Fluidwärmers auf. Die Steuereinrichtung ist dabei vorzugsweise ausgelegt, die ersten Schalter in Abhängigkeit von der Spannung zwischen dem Wärmeübertrager und dem Neutralleiter der Wechselspannungsquelle synchronisiert in ihren geöffneten Schaltzustand zu überführen. Im Fehlerfall, d.h., wenn beispielsweise eine mit der Fluidleitung elektrisch leitend verbundene Person, etwa aufgrund eines Gerätefehlers eines zeitgleich mit dem Fluidwärmer betriebenen weiteren Geräts, an Netzspannung anliegt (sogenannter "SFC"-Fehlerfall), kann dadurch ein für die Person potentiell gefahrvoller Ableitstrom, der von dem Fluid kapazitiv auf die Wärmebertragereinrichtung und von dieser (kapazitiv) auf die Widerstandsheizelemente übertragen und über den Neutralleiter der Wechselspannungsquelle abgeführt würde, unterbunden werden. Darüber hinaus kann auch einem vom Fluidwärmer herrührender unerwünschter Ableitstrom, beispielsweise in Folge einer Fehlfunktion des Fluidwärmers, frühzeitig erkannt und unterbunden werden. Dies ist für die elektrische Betriebssicherheit des Fluidwärmers insgesamt von Vorteil. Vorliegend wird unter dem geöffneten Schaltzustand der Schalter derjenige Schaltzustand verstanden, bei dem eine Stromleitung über die Schalter unterbrochen ist. Das vorgegebene Zeitintervall, innerhalb dessen die Schalter in ihren geöffneten Schaltzustand überführt werden, ist immer so bemessen, dass eine Gefährdung des Patienten durch Fehler- bzw. Patientenableitstöme sicher ausgeschlossen werden kann. Der erfindungsgemäße Fluidwärmer kann die einschlägige Norm für die internationale Sicherheitsklassifizierung "CF" (Kurzform für "Cardiac Floating") erfüllen, ohne dass hierzu eine separate Funktionserdung der Wärmeüberträgereinrichtung erforderlich ist. Dies bietet konstruktive Vorteile.

Die vorstehend erläuterte Kompensationsschaltung kann erfindungsgemäß auch bei der vorstehend erläuterten Beaufschlagung der Widerstandsheizelemente mit den zueinander gegenphasigen Wechselspannungen vorgesehen sein. Dadurch kann eine trotz des gegenphasigen Wechselspannungsbetrieb der Widerstandsheizelemente auftretende kapazitive Aufladung des Wärmeüberträgers durch die Einstrahlungen der Widerstandsheizelemente (und damit einhergehende unerwünschte Ableitströme) vollständig oder nahezu vollständig kompensiert werden. Dies kann beispielsweise bei nicht exakt aufeinander abgestimmten elektrischen Eigenschaften der Widerstandsheizelemente erforderlich sein.

Die Kompensationsschaltung kann insbesondere einen elektrischen LC-Schwingkreis mit einer Spule L und einem Kondensator C aufweisen, der an dem Wärmeübertrager angeschlossen ist. Der Kondensator und die Spule können dabei insbesondere in Reihe geschaltet sein. Die Spule kann im konstruktiv einfachsten Fall als Sekundärseite eines Transformators T ausgebildet sein, der primärseitig zum Widerstandsheizelement elektrisch parallel geschaltet ist. D.h., der Transformator ist primärseitig an die Netzanschlussleiter zumindest eines Widerstandsheizelements angeschlossen (d.h., zu dem Widerstandsheizelement parallel geschaltet) und kann somit zeitgleich mit den Widerstandsheizelementen an der Wechselspannungsquelle betrieben werden. Der LC-Schwingkreis kann dadurch zu (erzwungenen) elektrischen Schwingungen mit einer der Wechsel- bzw. Netzspannung entsprechenden Frequenz angeregt werden. Aufgrund der Phasenverschiebung des dadurch verursachten Stroms im LC-Schwingkreis kann der durch den zeitgleichen Betrieb des Widerstandsheizelements induzierte (kapazitive) Ableitstrom des Wärmeübertragers im Wesentlichen kompensiert werden. Die Gefahr einer möglichen Patientengefährdung wird dadurch verringert. Der oben beschriebene Schwingkreis kann auch mit elektronischen Bauelementen aufgebaut sein (z.B. unter Verwendung eines Mikroprozessors).

Die Kompensationsschaltung kann alternativ auch eine sogenannte Phasenregelschleife oder einen an sich bekannten Phasenschieber umfassen.

Der Fluidwärmer weist nach der Erfindung vorzugsweise eine Polungsumschalteinrichtung auf, mittels derer die Netzanschlussleiter mit einer vorgegebenen Polung mit der Wechselspannungsquelle verbindbar sind. Dadurch kann die Kompensation der kapazitiven Einstreuung der Widerspannungsheizelemente in den Wärmeüberträger ermöglicht werden, ohne dass hierzu eine weitere Kontrolle/eingangsseitige Umpolung der Polung der Kompensationsschaltung erforderlich ist.

Die Messeinrichtung kann insbesondere eine hardwaremäßig realisierte Komparatorschaltung aufweisen, mittels derer die elektrische Spannung zwischen dem Wärmeübertrager und dem Neutralleiter der Wechselspannungsquelle erfassbar ist.

Der Fluidwärmer weist für das Regeln des Temperiervorgangs vorzugsweise eine Temperaturmesseinrichtung mit einem dem Wärmeübertrager zugeordneten Temperatursensor auf. Der Temperatursensor ist vorzugsweise über eine elektrisch leitende Verbindungsleitung an eine Auswerte- und/oder Anzeigeeinheit angebunden. Die elektrische Betriebssicherheit des Fluidwärmers kann in diesem Fall erfindungsgemäß dadurch nochmals weiter erhöht werden, dass die elektrische Verbindungsleitung je Verbindungsleiter einen oder auch mehrere ansteuerbare Schalter aufweist, der/die, vorzugsweise durch die Steuereinrichtung, zeitgleich mit den ersten Schaltern der Anschlussleiter der Widerstandsheizelemente in ihren geöffneten Schaltzustand überführbar ist/sind.

Nach einer bevorzugten Weiterbildung der Erfindung ist der Wärmeübertrager funktionsgeerdet. Dadurch kann im Bereich des Wärmeübertrager, insbesondere bei einer nicht vollständigen Kompensation der kapazitiven Einstreuung in den Wärmeübertrager, zusätzlich ein örtlicher Potenzialausgleich geschaffen werden, ohne dass hierzu Ableitströme als sogenannte Patientenableitströme über das medizinische Fluid abfließen kann. Dadurch kann der Gefahr von Herzrhythmusstörungen oder auch Störungen von anderen medizintechnischen Apparaten und Geräten, wie beispielsweise einem EKG-Gerät oder einem internen/externen Defibrillator nochmals zuverlässiger entgegengewirkt werden.

Der Wärmeübertrager ist in diesem Fall mit einem Funktionserdungsleiter (FE) elektrisch leitend verbunden. Der Funktionserdungsleiter weist vorteilhaft einen, vorzugsweise von der Steuereinrichtung, ansteuerbaren dritten Schalter auf. Im Fehlerfall ist der Schalter des Funktionserdungsleiters gemeinsam (synchron) mit den ersten Schaltern der Anschlussleiter der Widerstandsheizelemente und, sofern vorhanden, mit dem zweiten Schalter der Temperaturmesseinrichtung, in den geöffneten Schaltzustand überführbar. Im Fehlerfall kann dadurch ein unerwünschtes Abfließen von gefahrvollen Fehlerströmen ("SFC"-Fehlerfall) über den Funktionserdungsleiter wirkungsvoll unterbunden werden.

Zu beachten ist, dass bei einer nicht vollständigen Kompensation der durch den Temperierbetrieb der Widerstandselemente im Wärmeübertrager kapazitiv induzierten Ableitströme über den Funktionserdungsleiter immer ein - wenn auch geringer - Ableitstrom fließt. Die Steuereinrichtung kann erfindungsgemäß einen Sensor zum Erfassen des Ableitstroms I_{A} über den Funktionserdungsleiter aufweisen. Die Steuereinrichtung ist dabei vorzugsweise programmiert, die funktionelle Integrität des Funktionserdungsleiters und/oder die Effizienz der Kompensationseinrichtung anhand der Größe bzw. des vorhandenen Ableitstroms zu überwachen. Ein Abfallen des über den Funktionserdungsleiter abgeleiteten Ableitstroms auf/unter einen vorgegebenen minimalen Schwellenstrom Iₘᵢₙ kann entweder auf eine schwerwiegende Funktionsstörung (Kurzschluss) eines mit dem Fluidwärmer zeitgleich an einer Person eingesetzten Geräts oder aber auf einen unterbrochenen Funktionserdungsleiter hinweisen. Der Funktionserdungsleiter ist aus messtechnischen Gründen vorzugsweise mit einem dem Sensor vorgeschalteten Vorschaltwiderstand versehen.

Die ansteuerbaren ersten, zweiten und/oder dritten Schalter können nach der Erfindung insbesondere als elektronische Schalter, beispielsweise als TRIACs (Zweirichtungs-Thyristortriode) oder als MOSFETs (Metall-Oxid-Halbleiter-Feldeffekttransistor), ausgebildet sein. Diese sind am Markt vorkonfektioniert und daher kostengünstig verfügbar. Im Falle der elektronischen Schalter können zudem besonders kurze Schaltzeiten realisiert werden. Aufgrund der fehlenden Schaltkontakte können dadurch überdies ein Verschleiß der Schalter sowie unerwünschte Prelleffekte vermieden werden. Die Schalter können nach einer Weiterbildung der Erfindung auch zumindest teilweise als Relais ausgebildet sein.

Das Zeitintervall, innerhalb dessen die Schalter in ihren geöffneten Schaltzustand überführbar sind, ist nach der Erfindung bevorzugt auf die Periodendauer der von der eingesetzten Wechselspannungsquelle bereitgestellten Wechselspannung ausgerichtet. Das Zeitintervall ist dabei in jedem Fall kürzer als eine Periodendauer T, bevorzugt kürzer als eine Halbperiodendauer T_{halb}, der von der Wechselspannungsquelle bereitgestellten Wechselspannung (=Netzspannung). Die maximale Schaltzeit der Schalter beträgt, beispielsweise bei einer Wechselspannung mit 50 Hz, vorzugsweise weniger als 10 ms, ganz besonders bevorzugt weniger als 1 ms.

Unter sicherheitstechnischen Aspekten hat es sich darüber hinaus als vorteilhaft erwiesen, wenn die Schalter jeweils doppelt, d.h., durch jeweils zwei in Serie angeordnete Einzelschalter, ausgeführt sind, die synchron betätigbar sind.

Die Schalter sind bevorzugt bei einer Spannung, die einem definierten Schwellenspannungswert entspricht oder größer als der Schwellenspannungswert ist, in ihren geöffneten Schaltzustand überführbar. Der Schwellenspannungswert kann insbesondere 132 Volt Wechselspannung betragen und ist vorzugsweise in der Steuereinrichtung abgespeichert.

Die Wärmeübertrager des Fluidwärmers kann im konstruktiv einfachsten Fall erfindungsgemäß zwei oder auch mehr Wärmeübertragerplatten aufweisen, die zwischen einander die Aufnahme für die Fluidleitung ausbilden. Die Wärmeübertragerplatten können dabei in an sich bekannter Weise gegeneinander bewegbar oder aber relativ zueinander starr an einem Wärmeplattenträger des Fluidwärmers angeordnet sein. Die Aufnahme kann beispielsweise einen Einschubkanal, für einen behälterartigen Leitungsabschnitt der Fluidleitung, wie beispielsweise einen Fluidbeutel oder eine Fluidkassette, sein. Dadurch wird ein großflächiger Kontakt des Wärmeübertragers mit der Fluidleitung und somit eine optimale Wärmeübertragung auf das darin strömende Fluid ermöglicht.

Im Hinblick auf ein effizientes Temperieren des medizinischen Fluids weist der Fluidwärmer vorzugsweise eine Abgabeleistung von zumindest 0,25 Kilowatt, bevorzugt von mehr als 0,35 Kilowatt, insbesondere ungefähr 0,4 Kilowatt, auf.

Das erfindungsgemäße Verfahren ermöglicht einen besonders zuverlässigen und sicheren Betrieb des erfindungsgemäßen Fluidwärmers.

Nachfolgend wird die Erfindung anhand eines in der Zeichnung wiedergegebenen Ausführungsbeispiels näher erläutert.

Die gezeigte und beschriebene Ausführungsformen sind nicht als abschließende Aufzählung zu verstehen, sondern haben für die Schilderung der Erfindung vielmehr beispielhaften Charakter.

In der Zeichnung zeigen:
- Fig. 1: ein Blockschaltbild eines erfindungsgemäßen Fluidwärmers zum Temperieren eines in einer Fluidleitung geführten Fluids, der zwei Widerstandsheizelemente mit Netzanschlussleitern für eine Wechselspannungsquelle und einen Wärmeübertrager aufweist, wobei die Netzanschlussleiter mit Schaltern versehen sind, die in Abhängigkeit von einer elektrischen Spannung zwischen dem Neutralleiter der Wechselspannungsquelle und dem Wärmeübertrager in ihren nicht-stromführenden Schaltzustand überführbar sind;
- Fig. 2: ein Blockschaltbild eines Fluidwärmers, bei dem der Wärmeüberträger zusätzlich funktionsgeerdet ist;
- Fig. 3: ein Schaltbild eines erfindungsgemäßen Verfahrens zum Betreiben der in den Figuren 1 und 2 gezeigten Fluidwärmer.

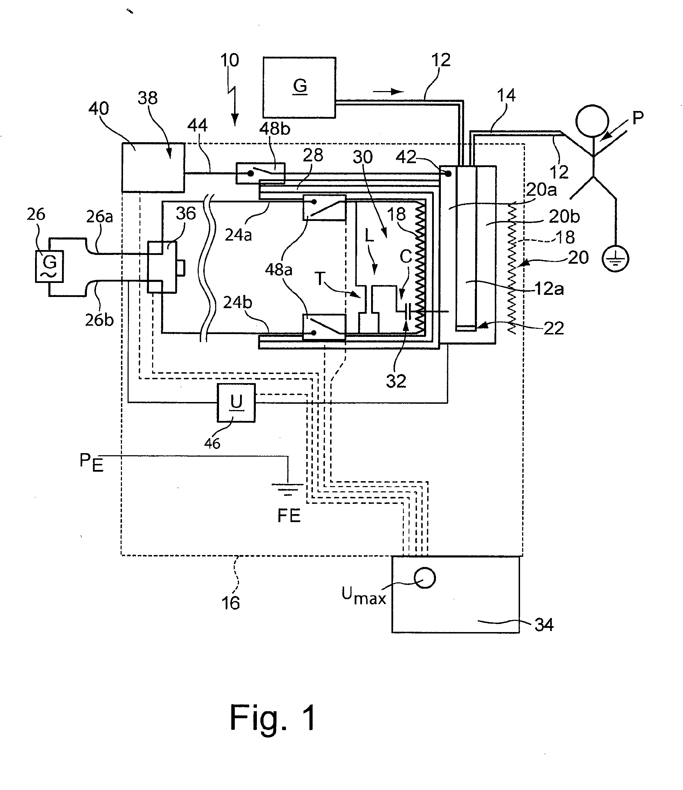

**Fig. 1** zeigt einen Fluidwärmer **10** zum Temperieren eines in einer Fluidleitung **12** geführten medizinischen Fluids **14.** Der Fluidwärmer 10 weist ein Gehäuse **16** mit zwei in dem Gehäuse **16** voneinander beabstandet angeordneten Widerstandsheizelementen **18** auf, die mit einem Wärmeübertrager **20** thermisch gekoppelt, d.h., mit diesem wärmeleitend verbunden, sind. Das in der Fig. 1 rechts dargestellte Widerstandsheizelement 18 ist aus Darstellungsgründen nur mit gestrichelter Linie angedeutet. Die Widerstandsheizelemente 18 sind vorliegend jeweils als Heizspirale ausgebildet, können aber auch eine andere Formgebung aufweisen.

Der Wärmeübertrager 20 weist zwei zueinander parallel ausgerichtete Wärmeübertragerplatten **20a, 20b** auf, die unter Ausbildung einer Aufnahme **22** voneinander beabstandet angeordnet sind. In der Aufnahme 22 ist ein beutelförmig erweiterter Leitungsabschnitt (Fluidbeutel) **12a** der Fluidleitung 12 eingeschoben bzw. eingelegt. Der Leitungsabschnitt 12a liegt an den beiden Wärmeübertragerplatten 20a, 20b des Wärmeübertragers 20 im wesentlichen vollflächig an. Dadurch wird eine optimale Wärmeübertragung auf das in der Fluidleitung strömend geführte Fluid 14 gewährleistet.

Die Fluidleitung 12 ist als eine im medizinischen Sektor an sich bekannte Infusionsleitung ausgebildet und besteht aus einem Kunststoff. Die Fluidleitung ist einenends, beispielsweise über einen nicht näher wiedergegebenen venösen Zugang /Venenverweilkatheter, an einen Blutkreislauf (nicht gezeigt) einer zu behandelnden Person **P,** angeschlossen. Das Fluid 14 steht somit vorliegend mit dem Blutkreislauf der Person P unter Umgehung des in der Regel hochohmigen Hautwiderstands in einem elektrisch leitfähigen Kontakt. Mit G ist ein weiteres (medizinisches) Gerät bezeichnet, das beispielsweise als Dialyseeinheit ausgebildet sein kann. Das Gerät G wird zeitgleich mit dem Fluidwärmer betrieben und ist mit diesem/der Person P über die Fluidleitung 12 verbunden.

Jedes Widerstandsheizelement 18 weist einen ersten und einen zweiten Netzanschlussleiter **24a, 24b** auf, die einem elektrischen Anschluss des Widerstandsheizelements 18 an eine Netzspannung, d.h, an eine externe Wechselspannungsquelle **26,** dienen. Die Netzanschlussleiter des in der Figur rechts dargestellten Widerstandsheizelements 18 sind nicht gezeigt.

Die für den Betrieb der Widerstandsheizelemente 18 vorgesehene Wechselspannungsquelle 26 weist eine übliche länderspezifische Betriebsspannung von beispielsweise 230 V ± 10%, und eine Netzfrequenz von ungefähr 50Hz auf.

Der in der Fig. obere Netzanschlussleiter 24a der Widerstandsheizelemente 18 ist vorliegend mit einem Außenleiter **26a** der Wechselspannungsquelle und der zweite Netzanschlussleiter 24b mit einem Neutralleiter **26b** der Wechselspannungsquelle 26 elektrisch leitend verbunden.

Die beiden Netzanschlussleiter 24a, 24b und das jeweilige Widerstandsheizelement 18 bilden einen Netzstromkreis des Fluidwärmers 10, der mittels einer verstärkten, hier doppelten, Isolierung **28** gegenüber dem Patienten P bzw. einem Anwender elektrisch isoliert ist. Bei dem in der Figur rechts dargestellten Widerstandsheizelement 18 ist die Isolierung 28 aus Darstellungsgründen nicht gezeigt.

An den beiden Widerstandsheizelementen 18 liegt bei dem gezeigten Ausführungsbeispiel im Temperierbetrieb des Fluidwärmers 10 jeweils eine Wechselspannung (=Netzspannung) an. Die beiden Wechselspannungen sind dabei zueinander gleichphasig, so dass die Widerstandsheizelemente 18 im Temperierbetrieb von zueinander gleichphasigen Heizströmen durchflossen sind.

Eine elektrische Kompensationseinrichtung, hier eine Kompensationsschaltung **30,** dient dazu, eine im Temperierbetrieb des Fluidwärmers 10 unvermeidbare kapazitive Einstreuung der Widerstandsheizelementen 18 in den Wärmeübertrager 20 zu kompensieren. Die Kompensationsschaltung 30 weist einen LC-Schwingkreis **32** mit einer Spule **L** und einem Kondensator **C** auf. Der LC-Schwingkreis ist an den Wärmeübertrager und den Netzanschlussleiter angeschlossen, der neutralleiterseitig an die Wechselspannungsquelle angeschlossenen ist. Die Spule L und der Kondensator C des LC-Schwingkreises 32 sind vorliegend in Reihe geschaltet. Die Spule L ist durch eine Sekundärseite eines Trafos **T** gebildet, der primärseitig an die beiden Netzanschlussleiter 24a, 24b der Widerstandsheizelemente 18 angeschlossen ist. Beim gemeinsamen Betrieb der Widerstandsheizelemente 18 und des Transformators T an der Netzspannung der Wechselspannungsquelle 26 wird der LC-Schwingkreis zu einer elektrischen Schwingung angeregt. Aufgrund einer (vorgegebenen) Phasenverschiebung des Stroms im LC-Schwingkreis (von ungefähr 180°) gegenüber dem Heizstrom der Widerstandsheizelemente 18 wird die kapazitive Einstreuung der Widerstandsheizelemente in den Wärmeübertrager 20 kompensiert oder im Wesentlichen kompensiert. Der oben beschriebene Schwingkreis kann auch mit elektronischen Bauelementen aufgebaut sein (z.B. unter Verwendung eines Mikroprozessors).

Eine Steuereinrichtung **34** mit einem nicht näher wiedergegebenen Anzeige- und Bedienfeld dient vorliegend einer Steuerung aller Betriebsparameter des Fluidwärmers 10. Die Steuereinrichtung 34 ist dazu programmiert, eine netzeingangsseitigen Polung der Netzanschlussleiter 24a, 24b an der Wechselspannungsquelle 26 zu detektieren. Die Polung der Netzanschlussleiter 24a, 24b kann mittels einer durch die Steuereinrichtung gesteuerten Polungsumschalteinrichtung **36** auf die in der Fig. 1 gezeigte (vorgegebene) Polung umgeschaltet werden. Dadurch ist die Kompensationsfunktion der Kompensationsschaltung unabhängig von einem mechanischen Anschluss der Netzanschlussleiter 24a, 24b gewährleistet.

Zur Temperaturüberwachung des Wärmeübertragers 20 dient eine Temperaturmesseinrichtung **38** mit einer Auswerteeinheit **40** und mit einem am oder im Wärmeübertrager 20 angeordneten Temperatursensor **42.** Der Temperatursensor 42 ist über eine elektrisch leitfähige Verbindungsleitung **44** mit der Auswerteeinheit 40 verbunden. Die Temperaturmesseinrichtung 38 kann Bestandteil der Steuereinrichtung 34 sein.

Die Steuereinrichtung 34 weist eine Spannungsmesseinrichtung **46** auf, mittels derer eine elektrische Spannung **U** zwischen dem Wärmeübertrager 20 und dem Neutralleiter 26b der Wechselspannungsquelle 26 erfasst bzw. überwacht werden kann.

Wie in Fig. 1 gezeigt ist, sind die beiden Netzanschlussleiter 24a, 24b der Widerstandsheizelemente 18 mit ansteuerbaren ersten Schaltern 48a und die Verbindungsleitung 44 der Temperaturmesseinrichtung 38 mit ansteuerbaren zweiten Schaltern 48b versehen. Die Schalter 48a, 48b sind jeweils zwischen einem eingeschalteten (geschlossenen) und einem in der Figur gezeigten ausgeschalteten (geöffneten) Schaltzustand umschaltbar. Im eingeschalteten Schaltzustand sind die Schalter 48a, 48b stromleitend, d.h., deren Schaltereingang ist mit dem Schalterausgang niederohmig elektrisch leitend verbunden. Im ausgeschalteten Schaltzustand sind die Schalter nicht stromleitend, d.h., deren Ein- und Ausgang sind im ausgeschalteten Schaltzustand jeweils voneinander elektrisch isoliert. Die Schalter 48a, 48b sind vorliegend jeweils als Relais ausgebildet, können aber auch als MOSFET (MetallOxid-Halbleiter-Feldeffekttransistor), als TRIAC (Zweirichtungs-Thyristortriode) oder dergl., ausgebildet sein. Die Schalter 48a, 48b sind von der Steuereinrichtung 34 ansteuerbar. Das vorgegebene Zeitintervall, innerhalb dessen die Schalter 48a, 48b durch die Steuereinrichtung 34 in ihren geöffneten Schaltzustand überführbar sind, ist immer kleiner als eine Periodendauer, vorzugsweise kleiner als eine Halbperiodendauer, der zum Betrieb der Widerstandsheizelemente 18 eingesetzten Wechselspannung der Wechselspannungsquelle 26. Das Zeitintervall ist bei einer Netzfrequenz von 50 Hz insbesondere kleiner als 10 ms.

In der Steuerungseinrichtung 34 ist für die elektrische Spannung zwischen dem Wärmeübertrager und dem Neutralleiter 26b der Wechselspannungsquelle 26 ein Schwellenspannungswert Uₘₐₓ hinterlegt (abgespeichert). Die Steuereinrichtung 34 ist dazu programmiert, die Schalter 48a, 48b in ihren geöffneten Schaltzustand zu überführen, sobald die Spannung U zwischen dem Wärmeübertrager und dem Neutralleiter 26b die Schwellenspannungswert Uₘₐₓ erreicht oder überschreitet. Dadurch kann im Fehlerfall (SFC) ein Abfließen eines unerwünschten Ableitstroms vom Wärmeübertrager 20 über die Widerstandsheizelemente 18 bzw. die Temperaturmesseinrichtung 38 und den Neutralleiter 26b der Wechselspannungsquelle 26 unterbunden werden. Darüber hinaus wird eine weitere kapazitive Einkopplung von den Widerstandsheizelementen 18 in den Wärmeüberträger 20 sowie das darin geführte Fluid 14 unterbunden. Einem unerwünschten Ableitstrom des Fluidwärmers über die Fluidleitung kann dadurch sicher entgegengewirkt werden.

Bei einem nicht näher gezeigten Ausführungsbeispiel des Fluidwärmers 10 liegen an den beiden Widerstandsheizelementen 18 im Temperierbetrieb zueinander gegenphasige Wechselspannungen an. In diesem Fall ist die vorstehend beschriebene Kompensationsschaltung nicht erforderlich. Zum Erzeugen einer zu der am einen Widerstandsheizelement 18 anliegenden Wechselspannung gegenphasigen Wechselspannung kann ein Phasenschieber oder dergl. eingesetzt werden.

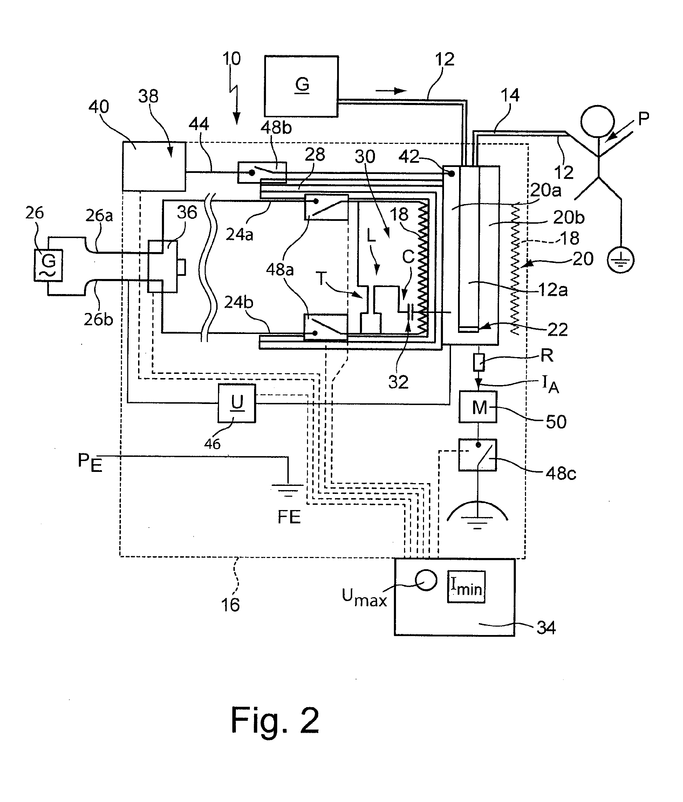

**Fig. 2** zeigt einen Fluidwärmer 10, der sich von dem im Zusammenhang mit Fig. 1 erläuterten Fluidwärmer 10 im Wesentlichen darin unterscheidet, dass der Wärmeübertrager 20 mittels eines Funktionserdungsleiters **FE** funktionsgeerdet ist. Der Funktionserdungsleiter FE ist mit dem Schutzleiter **PE** des Fluidwärmers 10 elektrisch leitend verbunden. Der Funktionserdungsleiter FE ist mit einem von der Steuereinrichtung ansteuerbaren dritten Schalter **48c** verbunden. Der ansteuerbare dritte Schalter kann zu den anderen Schaltern **48a, 48b** baugleich sein. Eine Strommesseinrichtung **50** ist mit der Steuerungseinrichtung 34 verbunden und dient dem Erfassen des über den Funktionserdungsleiter (ab)fließenden Ableitstroms 1. Der Strommesseinrichtung 50 ist ein Vorschaltwiderstand **R** vorgeschaltet. Im Temperierbetrieb des Fluidwärmers 10 wird bei einer nicht vollständigen Kompensation der kapazitiven Einstreuungen der Widerstandsheizelemente 18 in den Wärmeübertrager 20 grundsätzlich ein geringer Ableitstrom I_{A} über die Funktionserdungsleiter FE fließen. Die Steuereinrichtung 34 ist vorliegend dazu programmiert, den Ableitstrom I_{A} mit einer in der Steuereinrichtung 34 abgespeicherten minimalen Schwellenstromstärke Iₘᵢₙ zu vergleichen und, sofern der Ableitstrom I_{A} kleiner oder gleich der minimalen Schwellenstromstärke Iₘᵢₙ ist, die ansteuerbaren ersten, zweiten und dritten Schalter 38a, 38b, 38 c innerhalb der vorgegebenen Schaltzeit gemeinsam in ihren geöffneten Schaltzustand überführen.

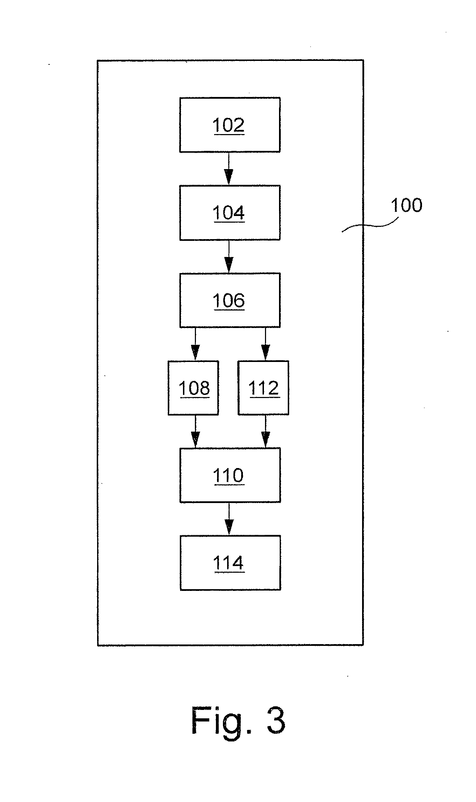

Nachfolgend wird das erfindungsgemäße Verfahren **100** zum Betrieb des Fluidwärmers 10 aus Fig. 1 und 2 unter zusätzlicher Bezugnahme auf **Fig.** 3 erläutert.

Die Steuereinrichtung 34 (Fig. 1, 2) des Fluidwärmers 10 ist zur Ausführung der einzelnen Verfahrensschritte programmiert. Die Steuereinrichtung 34 kann jedoch auch durch ihre rein hardwaremäßige Ausgestaltung die Durchführung des Verfahrens 100 ermöglichen.

Nach einem Anschließen **102** des Fluidwärmers 10 an die Wechselspannungsquelle 26 und einem Einschalten des Fluidwärmers 10 befindet sich die Steuereinrichtung 34 zunächst in einem Standby Modus.

Vor Beginn des Temperierbetriebs des Fluidwärmers 10 wird in einem weiteren Schritt **104** die Polung der Netzanschlussleiter 24a, 24b bezüglich der Wechselspannungsquelle 26 bestimmt und mit der vorgegebenen Polung vergleichen. Bei einer von der vorgegebenen Polung abweichenden Polung der Netzanschlussleiter 24a, 24b bezüglich des Außenleiters 26a und des Neutralleiters 26b der Wechselspannungsquelle 26 wird die Polung durch die Steuereinheit 34 mittels des Polungsumschalters 36 auf die in den Figuren 1 und 2 gezeigte vorgegebene Polung umgestellt.

Zur erstmaligen Aufnahme des Temperierbetriebs werden die ersten Schalter der beiden Netzanschlussleiter 24a, 24b in einem weiteren Schritt **106** geschlossen. Man beachte, dass für den Temperiervorgang das intermittierende "Ab"- und "Anschalten" der Widerstandsheizelemente 18 erforderlich ist. Dazu werden im Normalbetrieb des Fluidwärmers lediglich die ansteuerbaren ersten Schalter 48a der mit dem Außenleiter 26a der Wechselspannungsquelle 26 verbundenen Netzanschlussleiter 24a von der Steuereinrichtung 34 angesteuert.

Während des Temperierbetriebs des Fluidwärmers 10 wird in einem weiteren Schritt **108** die Spannung U zwischen dem Wärmeübertrager 20 und dem Neutralleiter 26b der Wechselspannungsquelle 26 von der Spannungsmesseinrichtung 46 kontinuierlich erfasst und die erfasste Spannung U von der Steuereinrichtung 34 mit dem in der Steuereinrichtung 34 hinterlegten Schwellenspannungswert Uₘₐₓ verglichen.

Sobald die Spannung U den Schwellenspannungswert Umax erreicht oder übersteigt, werden die Schalter **48a, 48b, 48c** in einem weiteren Schritt 110 innerhalb des vorgegebenen Zeitintervalls gemeinsam in ihren geöffneten Schaltzustand überführt, so dass sich der Fluidwärmer 10 in einem aktivierten Schutzmodus befindet.

Sofern der Fluidwärmer 10 mit einem funktionsgeerdeten Wärmeübertrager 20 versehen ist, überwacht die Steuereinrichtung im Temperierbetrieb des Fluidwärmers 10 in einem Schritt 112 zusätzlich den über den Funktionserdungsleiter FE fließenden Ableitstrom I_{A}. Sobald der Ableitstrom I_{A} gleich oder kleiner als die minimale Schwellenstromstärke Iₘᵢₙ ist, wird dies als eine Störung (Unterbrechung) des Funktionserdungsleiters bewertet und die Steuereinrichtung 34 überführt die Schalter 48a, 48b, 48c in dem Schritt **110** in ihren geöffneten Schaltzustand. Im stand-alone Betrieb des Fluidwärmers 10 kann so eine fehlerhafte Funktionserdung FE des Wärmeübertragers 20 festgestellt und eine Gefährdung des Patienten P durch unerwünscht hohe Patientenableitströme entgegengewirkt werden. Bei einem zeitgleichen Einsatz des Fluidwärmers 10 mit einem weiteren Gerät G am Patienten P können darüber hinaus Störungen des anderen Geräts G, bei dem kapazitive Ableitströme überwiegend oder alleinig über das andere Gerät G abgeführt werden, erkannt werden. Der Fluidwärmer 10 befindet sich auch hier in seinem aktivierten Schutzmodus. Der aktivierte Schutzmodus des Fluidwärmers 10 kann in einem weiteren Schritt 114 am Fluidwärmer 10 für eine Bedienperson durch einen Signalgeber optisch und/oder akustisch angezeigt werden.

## Patentansprüche

1. Fluidwärmer (10) zum Temperieren eines in einer Fluidleitung (12) geführten medizinischen Fluids (14), umfassend:
ein erstes und ein zweites Widerstandsheizelement (18) für Wechselspannung,
einen mit den beiden Widerstandsheizelementen (18) thermisch gekoppelten Wärmeübertrager (20), der eine Aufnahme (22) für die Fluidleitung (12, 12a) aufweist,
**dadurch gekennzeichnet, dass**:
- jedes Widerstandsheizelement (18) jeweils einen ersten und einen zweiten elektrischen Netzanschlussleiter (24a, 24b) zum Anschließen des Widerstandsheizelements (18) an eine Wechselspannungsquelle (26) aufweist,
- die Netzanschlussleiter (24a, 24b) der beiden Widerstandsheizelemente (18) jeweils mit einem ansteuerbaren ersten Schalter (48a) versehen sind;
- die Widerstandsheizelemente (18) im Temperierbetrieb jeweils mit einer zueinander gegenphasigen Wechselspannung beaufschlagt sind; oder
- die Widerstandsheizelemente (18) im Temperierbetrieb mit zueinander gleichphasigen Wechselspannungen beaufschlagt sind und der Wärmeübertrager (20) mittels einer Kompensationsschaltung (30) mit einer zur Wechselspannung im Wesentlichen gegenphasigen Kompensationswechselspannung beaufschlagt ist;
und ferner eine Spannungsmesseinrichtung (46) zum Bestimmen einer Spannung (U) zwischen dem Wärmeübertrager (20) und einem Neutralleiter (26b) der Wechselspannungsquelle (26); und
eine Steuereinrichtung (34) zum synchronisierten Überführen der Schalter (48a) in ihren geöffneten Schaltzustand in Abhängigkeit von der Spannung (U) vorgesehen sind

2. Fluidwärmer nach Anspruch 1, **dadurch gekennzeichnet, dass** die Kompensationsschaltung (30) einen LC-Schwingkreis (32) umfasst.

3. Fluidwärmer nach Anspruch 1, **dadurch gekennzeichnet, dass** die Kompensationsschaltung (30) eine Phasenregelschleife oder einen Phasenschieber umfasst.

4. Fluidwärmer nach einem der vorhergehenden Ansprüche, **dadurch gekennzeichnet, dass** der Fluidwärmer (10) eine Polungsumschalteinrichtung (36) zum Umschalten einer jeweiligen Polung der Anschlussleiter (24a, 24b) an der Wechselspannungsquelle (26) aufweist.

5. Fluidwärmer nach einem der vorhergehenden Ansprüche, **dadurch gekennzeichnet, dass** die Schalter (48a) bei einer Spannung (U), die gleich oder größer als ein in der Steuerungseinrichtung abgespeicherter definierter Schwellenspannungswert (Uₘₐₓ) ist, in ihren geöffneten Schaltzustand überführbar sind.

6. Fluidwärmer nach einem der vorhergehenden Ansprüche, **dadurch gekennzeichnet, dass** der Fluidwärmer eine Temperaturmesseinrichtung (38) mit einer Auswerteeinheit (40) und mit einem am oder im Wärmeübertrager (20; 20a, 20b) angeordneten Temperatursensor (42) aufweist, der über eine elektrische Verbindungsleitung (44) mit der Auswerteeinheit (40) verbunden ist, wobei
die Verbindungsleitung (44) mit einem ansteuerbaren zweiten Schalter (48b) versehen ist, der vorzugsweise synchron mit den ansteuerbaren ersten Schaltern (48a) der Netzanschlussleiter (24a, 24b), in seinen geöffneten Schaltzustand überführbar ist.

7. Fluidwärmer nach einem der vorhergehenden Ansprüche, **dadurch gekennzeichnet, dass** der Wärmeübertrager (20; 20a, 20b) mit einem Funktionserdungsleiter (FE) elektrisch leitend verbunden ist, wobei der Funktionserdungsleiter (FE) mit einem ansteuerbaren dritten Schalter (48c) versehen ist, der vorzugsweise synchron mit den ansteuerbaren ersten Schaltern (48a) der Netzanschlussleiter (24a, 24b) und/oder dem ansteuerbaren zweiten Schalter (48b) der Temperaturmesseinrichtung (38) in seinen geöffneten Schaltzustand überführbar ist.

8. Fluidwärmer nach Anspruch 7, **dadurch gekennzeichnet, dass** die Steuereinrichtung (34) eine Strommesseinrichtung (50) zum Erfassen eines Ableitstroms (I_{A}) über den Funktionserdungsleiter (FE) aufweist.

9. Fluidwärmer nach Anspruch 8, **dadurch gekennzeichnet, dass** die Steuereinrichtung (34) dazu programmiert ist, bei einem Ableitstrom (I_{A}) über den Funktionserdungsleiter (FE), der gleich oder kleiner als eine vorgegebene minimale Schwellenstromstärke (Iₘᵢₙ) ist, die ansteuerbaren ersten, zweiten und/oder dritten Schalter (48a, 48b, 48c), gemeinsam in den geöffneten Schaltzustand zu überführen.

10. Fluidwärmer nach einem der vorhergehenden Ansprüche, **dadurch gekennzeichnet, dass** zumindest ein Teil der ansteuerbaren ersten, zweiten und/oder der dritten Schalter (48a; 48b; 48c) Feldeffekttransistoren oder Relais sind.

11. Fluidwärmer nach einem der vorergehenden Ansprüche, **dadurch gekennzeichnet, dass** die Wärmeübertrager (20) zwei Wärmeübertragerplatten (20a,20b) aufweist, die zwischen einander die Aufnahme (22) für die Fluidleitung (12, 12a) ausbilden.

12. Verfahren (100) zum Steuern eines Fluidwärmers (10) nach einem der vorhergehenden Ansprüche, mit den folgenden Schritten:
- Erfassen (108) einer Spannung (U) zwischen dem Wärmeübertrager (20; 20a, 20b) und dem Neutralleiter (26b) der Wechselspannungsquelle (26);
- Überführen (110) der ansteuerbaren Schalter (48a, 48b, 48c) der Anschlussleiter (24a, 24b) der Widerstandsheizelemente in ihren geöffneten Schaltzustand, sofern die Spannung (U) gleich oder größer als ein vorgegebener Schwellenspannungswert ist.

## Claims

1. Fluid warmer (10) for tempering of a medical fluid (14) guided in a fluid line (12), comprising:
a first and a second resistance heating element (18) for alternating voltage,
a heat exchanger (20) which is thermally coupled to the two resistance heating elements (18) and comprises a holder (22) for the fluid line (12, 12a), **characterized in that**
- each resistance heating element (18) has a first and a second electrical line connector (24a, 24b), respectively, for connection of the resistance heating element (18) to an alternating voltage source (26),
- each line connector (24a, 24b), respectively, of the two resistance heating elements (18) is provided with a drivable first switch (48a);
- a respectively inversely phased alternating voltage is applied to each resistance heating element (18) during the tempering mode; or
- respectively in-phase alternating voltages are applied to the resistance heating elements (18) during the tempering mode and a compensation alternating voltage, which is substantially inversely phased with respect to the alternating voltage, is applied to the heat exchanger (20) using a compensation circuit (30);
and that there are moreover provided: a voltage measurement device (46) for determining a voltage (U) between the heat exchanger (20) and a neutral conductor (26b) of the alternating voltage source (26); and
a control device (34) for synchronized transfer of the switches (48a) into their open switching state in dependence on the voltage (U).

2. Fluid warmer according to claim 1, **characterized in that** the compensation circuit (30) comprises an LC resonant circuit (32).

3. Fluid warmer according to claim 1, **characterized in that** the compensation circuit (30) comprises a phase-locked loop or a phase shifter.

4. Fluid warmer according to any one of the preceding claims, **characterized in that** the fluid warmer (10) comprises a polarity switch (36) for switching a respective polarity of the line connectors (24a, 24b) at the alternating voltage source (26).

5. Fluid warmer according to any one of the preceding claims, **characterized in that** the switches (48a) are transferable into their open switching state at a voltage (U) which is equal to or larger than a defined threshold voltage value (Uₘₐₓ) stored in the control device.

6. Fluid warmer according to any one of the preceding claims, **characterized in that** the fluid warmer has a temperature measurement device (38) with an evaluation unit (40) and a temperature sensor (42) which is arranged on or in the heat exchanger (20; 20a, 20b) and is connected to the evaluation unit (40) via an electrical connecting line (44), wherein the connecting line (44) is provided with a drivable second switch (48b) which is transferable into its open switching state preferably synchronously with the drivable first switches (48a) of the line connectors (24a, 24b).

7. Fluid warmer according to any one of the preceding claims, **characterized in that** the heat exchanger (20; 20a, 20b) is electrically conductively connected to a functional grounding conductor (FE), wherein the functional grounding conductor (FE) is provided with a drivable third switch (48c), which is transferable into its open switching state preferably synchronously with the drivable first switches (48a) of the line connectors (24a, 24b) and/or the drivable second switch (48b) of the temperature measurement device (38).

8. Fluid warmer according to claim 7, **characterized in that** the control device (34) has a current measurement device (50) for detecting a leakage current (I_{A}) via the functional grounding conductor (FE).

9. Fluid warmer according to claim 8, **characterized in that** the control device (34) is programmed to jointly transfer the drivable first, second and/or third switches (48a; 48b; 48c) into the open switching state on occurrence of a leakage current (I_{A}) via the functional grounding conductor (FE), which is equal to or less than a defined minimum threshold current conduction (Iₘᵢₙ).

10. Fluid warmer according to any one of the preceding claims, **characterized in that** at least part of the drivable first, second and/or third switches (48a; 48b; 48c) are field-effect transistors or relays.

11. Fluid warmer according to any one of the preceding claims, **characterized in that** the heat exchanger (20) has two heat transfer plates (20a, 20b), forming the holder (22) for the fluid tube (12, 12a) therebetween.

12. Method (100) for controlling a fluid warmer (10) according to any one of the preceding claims, comprising the following steps:
- detecting (108) a voltage (U) between the heat exchanger (20; 20a, 20b) and the neutral conductor (26b) of the alternating voltage source (26);
- transferring (110) the drivable switches (48a, 48b, 48c) of the line connectors (24a, 24b) of the resistance heating elements in their open switching state provided that the voltage (U) is equal to or larger than a predetermined threshold voltage value.

## Revendications

1. Dispositif (10) de chauffage de fluide pour thermoréguler un fluide médical (14) guidé dans une conduite de fluide (12), comprenant :
un premier et un deuxième éléments (18) de chauffage par résistance pour tension alternative,
un échangeur de chaleur (20), qui est thermiquement couplé aux deux éléments (18) de chauffage par résistance et qui présente une partie réceptrice (22) pour la conduite de fluide (12, 12a),
**caractérisé en ce que**
- chaque élément (18) de chauffage par résistance présente respectivement un premier et un deuxième conducteurs électriques (24a, 24b) de raccordement au réseau, pour raccorder l'élément (18) de chauffage par résistance à une source (26) de tension alternative,
- les conducteurs (24a, 24b) de raccordement au réseau des deux éléments (18) de chauffage par résistance sont respectivement pourvus d'un premier commutateur asservissable (48a) ;
- les éléments (18) de chauffage par résistance sont, en mode de thermorégulation, respectivement sollicités par une tension alternative en opposition de phase par rapport à l' autre ; ou
- les éléments (18) de chauffage par résistance sont, en mode de thermorégulation, sollicités par des tensions alternatives de même phase l'une par rapport à l'autre, et l'échangeur de chaleur (20) est, au moyen d'un montage compensateur (30), sollicité par une tension alternative compensatrice essentiellement en opposition de phase par rapport à la tension alternative,
et **en ce qu'**il est en outre prévu
un équipement (46) de mesure de tension pour déterminer une tension électrique (U) entre l'échangeur de chaleur (20) et un conducteur neutre (26b) de la source (26) de tension alternative ; et
un équipement de commande (34) pour transférer de manière synchronisée les commutateurs (48a) dans leur état de commutation ouvert en fonction de la tension (U).

2. Dispositif de chauffage de fluide selon la revendication 1, **caractérisé en ce que** le montage compensateur (30) comprend un circuit oscillant (32) inductance-capacité.

3. Dispositif de chauffage de fluide selon la revendication 1, **caractérisé en ce que** le montage compensateur (30) comprend une boucle de régulation de phase ou un déphaseur.

4. Dispositif de chauffage de fluide selon l'une des revendications précédentes, **caractérisé en ce que** le dispositif (10) de chauffage de fluide présente un équipement (36) d'inversion de polarité pour inverser la polarité respective des conducteurs de raccordement (24a, 24b) à la source (26) de tension alternative.

5. Dispositif de chauffage de fluide selon l'une des revendications précédentes, **caractérisé en ce que** les commutateurs (48a) peuvent être transférés dans leur état de commutation ouvert à une tension (U) qui est supérieure ou égale à une valeur définie (Uₘₐₓ) de tension de seuil mémorisée dans l'équipement de commande.

6. Dispositif de chauffage de fluide selon l'une des revendications précédentes, **caractérisé en ce que** le dispositif de chauffage de fluide présente un équipement (38) de mesure de température avec une unité d'évaluation (40) et avec un capteur de température (42), qui est disposé sur ou dans l'échangeur de chaleur (20 ; 20a, 20b) et qui est relié à l'unité d'évaluation (40) par l'intermédiaire d'une ligne (44) de liaison électrique,
sachant que la ligne de liaison (44) est pourvue d'un deuxième commutateur asservissable (48b), qui peut être transféré dans son état de commutation ouvert de préférence en synchronisme avec les premiers commutateurs asservissables (48a) des conducteurs (24a, 24b) de raccordement au réseau.

7. Dispositif de chauffage de fluide selon l'une des revendications précédentes, **caractérisé en ce que** l'échangeur de chaleur (20 ; 20a, 20b) est relié en conduction électrique à un conducteur (FE) de mise à la terre fonctionnelle, sachant que le conducteur (FE) de mise à la terre fonctionnelle est pourvu d'un troisième commutateur asservissable (48c), qui peut être transféré dans son état de commutation ouvert de préférence en synchronisme avec les premiers commutateurs asservissables (48a) des conducteurs (24a, 24b) de raccordement au réseau et/ou avec le deuxième commutateur asservissable (48b) de l'équipement (38) de mesure de température.

8. Dispositif de chauffage de fluide selon la revendication 7, **caractérisé en ce que** l'équipement de commande (34) présente un équipement (50) de mesure de courant pour détecter un courant de fuite (I_{A}) passant par le conducteur (FE) de mise à la terre fonctionnelle.

9. Dispositif de chauffage de fluide selon la revendication 8, **caractérisé en ce que** l'équipement de commande (34) est programmé pour transférer conjointement dans l'état de commutation ouvert les premiers, deuxième et/ou troisième commutateurs asservissablés (48a, 48b, 48c) en présence d'un courant de fuite (I_{A}) passant par le conducteur (FE) de mise à la terre fonctionnelle qui est inférieur ou égal à une intensité minimale prédéfinie (Iₘᵢₙ) de courant de seuil.

10. Dispositif de chauffage de fluide selon l'une des revendications précédentes, **caractérisé en ce qu'**au moins une partie des premiers, deuxième et/ou troisième commutateurs asservissables (48a ; 48b ; 48c) sont des relais ou des transistors à effet de champ.

11. Dispositif de chauffage de fluide selon l'une des revendications précédentes, **caractérisé en ce que** l'échangeur de chaleur (20) comprend deux plaques (20a, 20b) d'échangeur de chaleur qui forment entre elles la partie réceptrice (22) pour la conduite de fluide (12, 12a).

12. Procédé (100) pour commander un dispositif (10) de chauffage de fluide selon l'une des revendications précédentes, avec les étapes suivantes :
- détection (108) d'une tension électrique (U) entre l'échangeur de chaleur (20 ; 20a, 20b) et le conducteur neutre (26b) de la source (26) de tension alternative ;
- transfert (110) des commutateurs asservissables (48a, 48b, 48c) des conducteurs de raccordement (24a, 24b) des éléments de chauffage par résistance dans leur. état de commutation ouvert dans la mesure où la tension (U) est supérieure ou égale à une valeur prédéfinie de tension de seuil.
